# EUROPEAN PATENT SPECIFICATION

(11) **EP 1 449 488 B1**
(45) Date of publication and mention of the grant of the patent: **07.11.2007**
(21) Application number: 03251076.0
(22) Date of filing: 21.02.2003
(51) Int. Cl.: A61B 18/14

(54) **Resectoscope electrode and method of fabricating the same**
Elektrode für ein Resektoskop und Herstellungsverfahren dafür
Electrode pour une resectoscope et méthode de fabrication correspondant

(43) Date of publication of application: 25.08.2004
(73) Proprietor: Unirose Medical Ltd., Hayes, Middlesex UB3 1ST (GB)
(72) Inventor: Bharj, Kamaljit Singh Unirose Medical Ltd Unit 6, Hayes UB6 1ST, Middlesex (GB)
(74) Representative: Lamb, Martin John Carstairs

(56) References cited:
- US-A- 4 149 538
- US-A- 4 917 082

## Description

The present invention relates to a resectoscope electrode, and a method of making a resectoscope electrode. The inventions relates particularly to a disposable high frequency resectoscope electrode.

Resectoscopes are well known in the surgical art, particularly in the field of urology. A resectoscope comprises a longitudinally extending optical cable with a cutting means at one end and a vision device at the other end to allow an operator to see the action of the cutting means. Resectoscopes are very well known and will not be described further here.

The cutting means may be a blade but is more commonly an electrical device called a resectoscope electrode. In use, electrical current is supplied to the resectoscope electrode to heat up an electrical element leading to cutting by the heating action of the element. Typically an electrical circuit is formed through the body of the patient. A known type of resectoscope electrode assembly is described for example in WO96/37156 It describes a resectoscope electrode assembly for giving simultaneous cutting and coagulation. It is supplied with electrical power at some frequency within the radio frequency range.

Whilst it is known to supply electrical power to the resectoscope as alternating current at frequencies within the radio frequency range, it is particularly preferred to supply electrical power in a range known in the art of electrosurgical implements as the high frequency range, which is generally above 300khz and preferably above 350khz. This gives particularly advantageous cutting or coagulation effect.

Typically, resectoscope electrodes have been provided in the past for multiple use. This has been case, because prior art resectoscope electrodes have been relatively expensive and cannot be disposed of after a single use. However, repeated use of the resectoscope electrode also gives problems. In the first place, it can be very difficult to completely sterilise the electrode after use, leading to the risk of cross infection between patients. Secondly, the cutting element of a resectoscope electrode may be a very thin wire which is vulnerable to breakage. The more times a resectoscope electrode is used, the greater the likelihood of the wire breaking inside the patient, with attendant problems.

US4149538 provides a design of resectoscope electrode assembly in which various parts of the resectoscope electrode are inserted into a non-conducting bearing tube and then fixed in position using adhesive or sealing compound. It is indicated that this design can be constructed sufficiently economically to allow it to be a single use resectoscope electrode. However, a high frequency resectoscope electrode requires particularly high levels of electrical insulation and physical stability. US4149538 does not disclose a method of making a resectoscope electrode assembly for use with high frequency power sources which is sufficiently cheap to construct to allow it to be a single use item.

US 4917082 discloses a resectoscope electrode according to the preamble of independent claim 1.

The present invention provides a disposable high frequency resectoscope electrode, usable for cutting or coagulating tissue, comprising:
an electrically conducting tissue cutting or coagulating member, supported at each end by an arm,
a longitudinally extending electrical conductor for supplying electrical current to each arm ;
a longitudinally extending insulating sheath formed around the longitudinally extending electrical conductor
a junction where the arms are electrically connected to the longitudinally extending conductor, characterised in that the junction is integrally moulded with the longitudinally extending insulating sheath formed around the longitudinally extending electrical conductor.

The present invention further provides a method of manufacturing a resectoscope electrode, comprising the steps of assembling an electrically conductive tissue cutting or coagulating member with arms at each end for supporting the tissue cutting or coagulating member, and a longitudinally extending electrical conductor, forming an electrical junction between the electrical conductor (4) and the arms and forming a thermoplastic body by in situ moulding around the junction of the arms and the longitudinally extending electrically conductor, characterised by the step of integrally moulding the junction with a longitudinally extending insulating sheath formed around the longitudinally extending electrical conductor.

In the invention, the insulating sheath of the longitudinally extending electrical conductor and the junction are integral with one another and are formed in the same moulding-step. This has particular advantages in a giving a strong structure. Further, a large part of the resectoscope electrode is formed in a single step, giving economy in design and construction.

The resectoscope electrode of the invention is suitably designed so that it is compatible with any conventional or commercially available resectoscope equipment. The present invention thus provides an accessory for a resectoscope system. The skilled person will be able to construct the resectoscope electrode of the present invention in such a way that it meets any statutory requirements, such as the Medical Device Classification and the relevant European directives.

The resectoscope electrode of the present invention is preferably supplied sterile. It is preferably in a sterile package.

The present invention accordingly further provides a sterile package comprising a sterile disposal high frequency resectoscope electrode

The resectoscope electrode of the present invention is preferably assembled in a clean room environment, to further improve the sterile properties of the electrode.

In order to be supplied sterile, the resectoscope electrode is suitably formed of materials which will resist the sterilisation process. The sterilisation process may be any suitable process, but preferably comprises gamma radiation sterilisation. The materials should then be gamma radiation resistant.

By 'high frequency' it is meant that the resectoscope electrode should be usable with a source of alternating electrical current with a frequency above 300khz and preferably above 350 kHz. Preferably, the resectoscope electrode can be used at up to 10Mhz, preferably up to 1 Mhz.

The electrically conducting tissue cutting coagulating member may have any suitable configuration. Many configurations are well known in the art and any of them can be used for the present invention. For example, the member may comprise a thin loop of metal, preferably tungsten having an angled, straight or pointed or other configuration. The member may comprise a coagulating member. The coagulating member may be relatively thick, and may for example have a ball or cylinder shape, preferably of diameter in the range 1-6mm preferably 3-5mm. It may be conical in form.

The arms have any suitable configuration to hold the respective ends of the tissue cutting or coagulating member apart. For example, the arms may diverge from the junction or they may run parallel from the junction to a diverging point, then diverge and then run parallel to one another.

The arms may be of any suitable construction. For example, they may comprise an electrical conductor surrounded by insulating material, the electrical conductor being connected at its ends to the tissue cutting or coagulating member. The electrical conductor may be continuous and integral with the element. The arms may further comprise rigid structures, for example tubes. Tubes may be made of any rigid material, for example thermoplastic or metal, particularly preferably steel. The tubes may surround the electrical conductor and be themselves surrounded with insulating material or they may surround a layer of insulating material formed around the conductor. In a particularly preferred embodiment, the arms are connected by a fixing clip. Preferably, the fixing clip is formed adjacent the junction. This gives additional strength to the structure. Because the item is for single use only, recesses formed by the clip which could otherwise form a trap for material which could lead to cross infection, are not a problem. The clip is preferably formed of steel.

The longitudinally extending electrical conductor may be of any suitable design, as is well known in the art. A rigid stabilising member may be provided to give strength to the electrical conductor. For example, a rigid tube may be provided surrounding the electrical conductor and the sheath or it may itself be surrounded by the insulating sheath. The rigid tube may be formed of thermoplastic or metal, for example steel.

The junction between the arms and the longitudinally extending electrical conductor is important in the present invention. The junction is preferably a moulded thermoplastic body which is moulded in situ. This has many advantages. In the first place, it forms a particularly strong connection between the electrical conductor and the arms. It can insulate the electrically conducting parts of the arms and electrical conductor against other conducting parts, for example the resectoscope itself or the patient's body, avoiding short circuits or electrical shocks. It is found that moulding the entire junction in situ can give a particularly strong structure.

The thermoplastic material of which the junction is made is suitably gamma radiation resistant, so that the electrode may be sterilised by gamma radiation.

Many prior art re-usable electrodes are designed so that they may be autoclaved after use. However, as the present invention is intended as a single use item, it need not be formed of autoclavable material.

It is also possible in this way to make a design in which the component parts are assembled in the correct configuration and then fixed to one another in the moulding steps. By forming the fixing in the in situ molding step, fixing can be carried out in substantially one step, leading to a particularly economic construction which allows the whole device to be sufficiently cheap to be reusable. Preferably, the in situ moulding step is an injection moulding step.

The thermoplastic material used to form a junction has to be carefully selected. It must be capable of being injection moulded under conditions and temperatures which will not damage the component parts of the resectoscope electrode. When formed, it must be strong but not brittle. It must be compatible with the human body. It must have the required electrical insulating properties.

The present inventors have discovered that polyetherketones are particularly preferred. It is particularly preferred that the material comprises poly (1,4-oxyphenylene carbonyl - 1,4-phenylene). This material is known as PEEK. Suitable material is available from Other injection mouldable materials may be used, for example LDPE, HDPE, polypropylene, ethylyinyl acetate, ionomer resin, PVC, styrene-ethylene butylenes-styrene, polyamide, polyether block amide, polyurethane, polycarbonate, polysulphone, polyoxymethylene, or polyethylene/butylenes terephthalate.

The resectoscope electrode of the present invention may be used with a standard design of resectoscope assembly in a manner which is known in the art. In order to allow the resectoscope electrode to be mounted on a resectoscope, there is preferably a mounting part formed on the resectoscope electrode. In a particularly preferred embodiment, the mounting is integrally moulded with the junction.

Preferably, the resectoscope system of the present invention is assembled in a sterile or clean room environment to prevent infection during use of the system.

The present invention will be further described by way of example with reference to the accompanying drawings, in which:
Figure 1 is sketch isometric view of a resectoscope electrode which is not according to the invention but is shown for reference.
Figure 2 is a sketch isometric view of an embodiment of resectoscope electrode according to the invention.
Figure 3 is a schematic, enlarged cross-sectional view of the junction of the resectoscope electrode of the Figure 1.
Figure 4 is a sketch isometric underside view at much enlarged scale of the clip shown in figures 1 and 2.

### DETAILED DESCRIPTION OF THE DRAWINGS

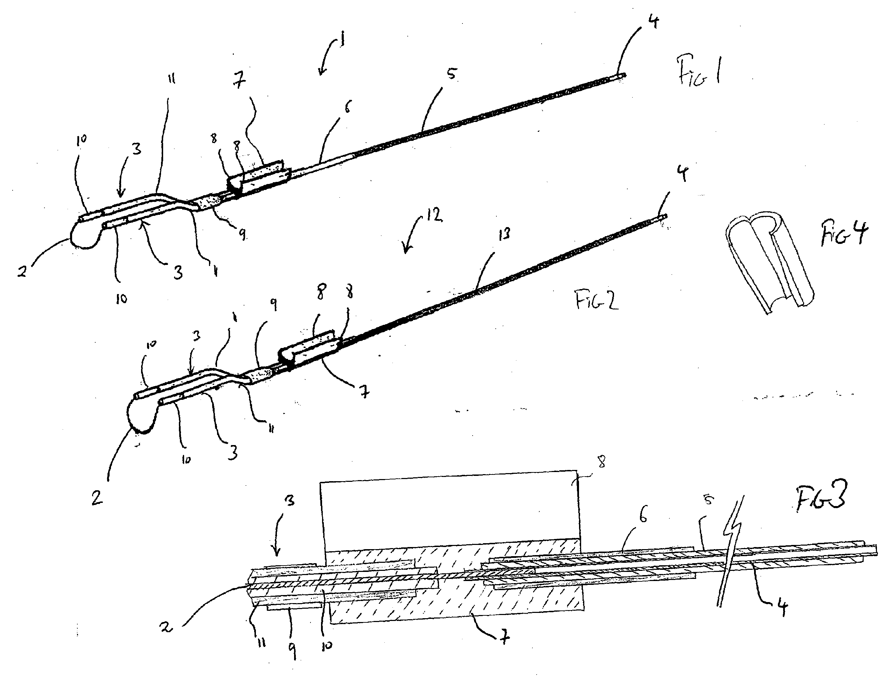

Figure 1 shows a sketch isometric view of a resectoscope electrode which is not according to the invention but which is described for reference.

The electrode 1 comprises an electrically conducting tissue cutting member a form of a tungsten wire element 2. The element 2 is supported at each end by an arm 3. The element is in the form of a cutting loop. It is to be understood that the present invention may be used with any configuration of cutting or coagulating member as is known in the art. The electrode further comprises a longitudinally extending electrical conductor 4 which can be seen at the end of the electrode. The conductor is sheathed along the greater part of its length in an insulating material 5, in this case formed of PTFE. The insulating material itself is covered at the end nearest the element 2 with a stainless steel tube 6 to add rigidity. At the junction where the longitudinally extending electrical conductor 4, the sheath in its insulator 5 and tube 6 meet the arm 3, there is an in situ moulded thermoplastic junction 7 formed of PEEK. The junction comprises an integrally molded mounting for fitting the resectoscope electrode to a resectoscope assembly, the mounting comprising a pair of upstanding wings 8 for forming a resilient clip - on structure.

A metal stabilising clip 9 is provided rigidly connecting the arms 3 together adjacent the junction, to give added strength. It can be seen that each arm 3 comprises an insulating tube 10, formed of PTFE which is contained inside a metal tube 11, to give additional strength. The metal tube 11 is made of stainless steel.

The resectoscope electrode 12 of the invention is generally similar in construction to the resectoscope electrode 1 of figure 1 and like parts are given the same reference numerals and will not be described further here.

However, the longitudinally extending electrical conductor 4 does not have a separate insulating sheath 5 and steel tube 6. Instead, there is an integrally molded sheath 13 formed of PEEK which is formed in the same moulding step as the junction 7. Figure 3 shows a sketch cross-section through the junction 7 of the resectoscope electrode 1 of figure 1. It is shown that in larger scale, but is not necessarily to scale.

It can be seen that the metal wire which forms the element 2 extends along the arm 3 inside the PTFE insulating tube 10 and inside the rigid steel tube 11 up to the junction 7. The clip 9 can also be seen. The PTFE insulating tube 10 and steel tube 11 stop short of the end of the wire 2 so that a part of it is left exposed to the end. This part is inserted into the end of the longitudinal electrical conductor 4 which is formed in a tubular shape which makes a close fit with the wire 2, to ensure electrical contact. The end of the tube 4 may be crimped onto the wire 2 to improve electrical contact, during manufacture. The insulating sheath 5 and steel tube 6 can also be seen.

In order to give physical support to the junction between the wire 2 inside the arm 3 and the longitudinally extending electrical conductor 4, the junction is contained in an integrally moulded thermoplastic in situ molding 7 composed of PEEK. One wing of the clip on moulding 8 for the resectoscope can be seen. The moulding 7 also provides extra insulation at the junction to prevent a short circuit or an electric shock for a user or the patient.

The clip 9 is shown at enlarged scale in figure 4. It can be seen that it comprises a bent steel strip which can be crimped into place over the arms 3 to hold them rigidly together.

The resectoscope electrode can be used with electrical power sources operating in the high frequency range. In practice this means 300khz-10Mhz, preferably 350khz-1Mhz. The electrode has been tested successfully at 400khz and 800khz.

The resectoscope electrode is formed entirely of materials that can be sterilised using gamma ray radiation. The assembly of the resectoscope electrode is carried out under sterile conditions in a clean room. Following assembly, the resectoscope electrode is sterilised using gamma radiation and sealed in a sealed sterile package.

## Claims

1. A disposable high frequency resectoscope electrode (12), usable for cutting or coagulating tissue, comprising:
an electrically conducting tissue cutting or coagulating member (2), supported at each end by an arm (3);
a longitudinally extending electrical conductor (4) for supplying electrical current to each arm (3);
a longitudinally extending insulating sheath (13) formed around the longitudinally extending electrical conductor (4);
a junction (7) where the arms (3) are electrically connected to the longitudinally extending conductor (4), **characterised in that** the junction (7) is integrally moulded with the longitudinally extending insulating sheath (13) formed around the longitudinally extending electrical conductor (4).

2. The resectoscope electrode of claim 1, wherein, at the junction (7), the arms (3) and the longitudinally extending conductor (4) are contained in a thermoplastic body (7) which is moulded in situ.

3. The resectoscope electrode according to claim 2, wherein the thermoplastic body (7) is injection moulded.

4. The resectoscope electrode according to claim 2 or 3, wherein the moulded body (7) comprises a polyetherketone.

5. The resectoscope electrode of any of claims 2 to 4, wherein the molded body (7) comprises poly (1,4-oxyphenylene carbonyl-1,4- phenylene).

6. The resectoscope electrode of any of claims 1 to 4, wherein the arms (3) are connected by a fixing clip (9) adjacent to the junction (7).

7. The resectoscope electrode of any preceding claim, comprising a mounting (8) for a resectoscope, the mounting being integrally moulded with the junction (7).

8. The resectoscope electrode of any preceding claim, contained in a sterile package.

9. The resectoscope electrode of any preceding claim, assembled in a clean room environment.

10. The resectoscope electrode of any preceding claim, comprising gamma ray resistant materials.

11. A sterile package comprising a resectoscope electrode according to any preceding claim.

12. A method of manufacturing a resectoscope electrode (12), comprising the steps of assembling an electrically conductive tissue cutting or coagulating member (2) with arms (3) at each end for supporting the tissue cutting or coagulating member (12), and a longitudinally extending electrical conductor (4), forming an electrical junction between the electrical conductor (4) and the arms (3) and forming a thermoplastic body (7) by in situ moulding around the junction (7) of the arms (3) and the longitudinally extending electrically conductor (4), **characterised by** the step of integrally moulding the junction (7) with a longitudinally extending insulating sheath (13) formed around the longitudinally extending electrical conductor (4).

## Patentansprüche

1. Hochfrequenz-Resektoskopelektrode zum einmaligen Gebrauch (12), die zum Schneiden oder Koagulieren von Gewebe verwendbar ist, umfassend:
ein elektrisch leitendes Gewebeschneid- oder -koagulationsteil (2), das an jedem Ende von einem Arm (3) gehalten wird;
einen sich in Längsrichtung erstreckenden elektrischen Leiter (4) zur Zuführung von elektrischem Strom zu jedem Arm (3);
eine sich in Längsrichtung erstreckende Isolierhülle (13), die um den sich in Längsrichtung erstreckenden elektrischen Leiter (4) ausgebildet ist;
eine Verbindung (7), wo die Arme (3) mit dem sich in Längsrichtung erstreckenden Leiter (4) elektrisch verbunden sind, **dadurch gekennzeichnet, daß** die Verbindung (7) mit der sich in Längsrichtung erstreckenden Isolierhülle (13), die um den sich in Längsrichtung erstreckenden elektrischen Leiter (4) ausgebildet ist, einstückig geformt ist.

2. Resektoskopelektrode nach Anspruch 1, wobei an der Verbindung (7) die Arme (3) und der sich in Längsrichtung erstreckende Leiter (4) in einem thermoplastischen Körper (7) enthalten sind, der an Ort und Stelle geformt ist.

3. Resektoskopelektrode nach Anspruch 2, wobei der thermoplastische Körper (7) spritzgegossen ist.

4. Resektoskopelektrode nach Anspruch 2 oder 3, wobei der geformte Körper (7) ein Polyetherketon aufweist.

5. Resektoskopelektrode nach einem der Ansprüche 2 bis 4, wobei der geformte Körper (7) Poly(1,4-Oxyphenylencarbonyl-1,4-Phenylen) aufweist.

6. Resektoskopelektrode nach einem der Ansprüche 1 bis 4, wobei die Arme (3) durch eine Befestigungsklammer (9) nahe der Verbindung (7) verbunden sind.

7. Resektoskopelektrode nach einem der vorhergehenden Ansprüche, umfassend eine Halterung (8) für ein Resektoskop, wobei die Halterung mit der Verbindung (7) einstückig geformt ist.

8. Resektoskopelektrode nach einem der vorhergehenden Ansprüche, die in einer sterilen Verpackung enthalten ist.

9. Resektoskopelektrode nach einem der vorhergehenden Ansprüche, die in einer Reinraum-Umgebung montiert ist.

10. Resektoskopelektrode nach einem der vorhergehenden Ansprüche, die gammastrahlenbeständige Materialien aufweist.

11. Sterile Verpackung, umfassend eine Resektoskopelektrode nach einem der vorhergehenden Ansprüche.

12. Verfahren zur Herstellung einer Resektoskopelektrode (12) umfassend die folgenden Schritte: Montieren eines elektrisch leitfähigen Gewebeschneid- oder -koagulationsteils (2) mit Armen (3) an jedem Ende zum Halten des Gewebeschneid- oder -koagulationsteils (2) und eines sich in Längsrichtung erstreckenden elektrischen Leiters (4), Ausbilden einer elektrischen Verbindung zwischen dem elektrischen Leiter (4) und den Armen (3) und Ausbilden eines thermoplastischen Körpers (7) durch an Ort und Stelle erfolgendes Formen um die Verbindung (7) der Arme (3) und des sich in Längsrichtung erstreckenden elektrischen Leiters (4), **gekennzeichnet durch** den folgenden Schritt: einstückiges Formen der Verbindung (7) mit einer sich in Längsrichtung erstreckenden Isolierhülle (13), die um den sich in Längsrichtung erstreckenden elektrischen Leiter (4) ausgebildet ist.

## Revendications

1. Electrode de resectoscope haute fréquence jetable (12), utilisable pour découper ou coaguler un tissu, comprenant:
un élément de découpe ou de coagulation (2) de tissu électriquement conducteur, supporté à chaque extrémité par un bras (3);
un conducteur électrique s'étendant longitudinalement (4) pour fournir un courant électrique à chaque bras (3);
une gaine isolante s'étendant longitudinalement (13) formée autour du conducteur électrique s'étendant longitudinalement (4);
une jonction (7) où les bras (3) sont raccordés électriquement au conducteur s'étendant longitudinalement (4), **caractérisé en ce que** la jonction (7) est moulée intégralement avec la gaine isolante s'étendant longitudinalement (13) formée autour du conducteur électrique s'étendant longitudinalement.

2. Electrode de resectoscope selon la revendication 1, dans lequel, sur la jonction (7), les bras (3) et le conducteur s'étendant longitudinalement (4) sont contenus dans un corps thermoplastique (7) qui est moulé in situ.

3. Electrode de resectoscope selon la revendication 2, dans lequel le corps thermoplastique (7) est moulé par injection.

4. Electrode de resectoscope selon la revendication 2 ou 3, dans lequel le corps moulé (7) comprend un polyéthercétone.

5. Electrode de resectoscope selon l'une quelconque des revendications 2 à 4, dans lequel le corps moulé (7) comprend du poly(1,4-oxyphénylènecarbonyl-1,4-phénylène).

6. Electrode de resectoscope selon l'une quelconque des revendications 1 à 4, dans lequel les bras (3) sont raccordés par un clip de fixation (9) adjacent à la jonction (7).

7. Electrode de resectoscope selon une revendication précédente quelconque, comprenant un montage (8) pour un resectoscope, le montage étant moulé intégralement avec la jonction (7).

8. Electrode de resectoscope selon une revendication précédente quelconque, contenue dans un emballage stérile.

9. Electrode de resectoscope selon une revendication précédente quelconque, assemblée dans un environnement de salle blanche.

10. Electrode de resectoscope selon une revendication précédente quelconque, comprenant des matériaux résistant aux rayons gamma.

11. Emballage stérile comprenant une électrode de resectoscope selon l'une quelconque des revendications précédentes.

12. Procédé de fabrication d'une électrode de resectoscope (12), comprenant les étapes d'assemblage d'un élément de découpe ou de coagulation de tissu électriquement conducteur (2) avec des bras (3) à chaque extrémité pour supporter l'élément de découpe et de coagulation de tissu (2), et un conducteur électrique s'étendant longitudinalement (4), formant une jonction électrique entre le conducteur électrique (4) et les bras (3) et formant un corps thermoplastique (7) par un moulage in situ autour de la jonction (7) des bras (3) et le conducteur électrique s'étendant longitudinalement (4), **caractérisé par** l'étape de moulage de façon intégrale de la jonction (7) avec une gaine isolante s'étendant longitudinalement (13) formée autour du conducteur électrique s'étendant longitudinalement.
